# EUROPÄISCHE PATENTSCHRIFT

(11) **EP 3 185 847 B1**
(45) Veröffentlichungstag und Bekanntmachung des Hinweises auf die Patenterteilung: **16.02.2022**
(21) Anmeldenummer: 15756146.5
(22) Anmeldetag: 25.08.2015
(51) Int. Cl.: A61K 8/365, A61Q 5/00, A61Q 5/02, A61K 8/19

(54) **VERFAHREN ZUR ERHÖHUNG DER WASCHBESTÄNDIGKEIT KÜNSTLICH GEFÄRBTER HAARE**
METHOD FOR INCREASING THE WASH RESISTANCE OF ARTIFICIALLY COLOURED HAIR
PROCÉDÉ POUR AUGMENTER LA RÉSISTANCE AU LAVAGE DES CHEVEUX ARTIFICIELLEMENT COLORÉS

(30) Priorität: 29.08.2014 DE 102014217310
(43) Veröffentlichungstag der Anmeldung: 05.07.2017
(73) Patentinhaber: Henkel AG & Co. KGaA, 40589 Düsseldorf (DE)
(72) Erfinder: SCHULZE ZUR WIESCHE, Erik, 22453 Hamburg (DE); KROHN, René, 22851 Norderstedt (DE)
(86) Internationale Anmeldenummer: PCT/EP2015/069403
(87) Internationale Veröffentlichungsnummer: WO 2016/030353

(56) Entgegenhaltungen:
- EP-A1- 1 118 319
- WO-A1-2012/084903
- WO-A2-02/32386
- JP-A- 2009 007 283

## Beschreibung

Die Erfindung liegt auf dem Gebiet der Kosmetik und betrifft ein Verfahren zur Verringerung und/oder Vermeidung des Ausblutens und/oder Verblassens künstlich erzeugter Haarfarben, umfassend die Aufbringung einer speziellen kosmetischen Zusammensetzung auf die Haare.

Die Erfindung betrifft weiterhin eine kosmetische Reinigungs- und/oder Pflegezusammensetzung zur Verringerung und/oder Vermeidung des Ausblutens und/oder Verblassens künstlich erzeugter Haarfarben, die neben einem anionischen Calciumlactat und/oder Milchsäure und Calciumchlorid und/oder Calciumhydroxid enthält.

Produkte zur Veränderung der natürlichen Haarfarbe spielen in der Haarkosmetik eine herausragende Rolle. Man unterscheidet permanente, semipermanente oder temporäre Färbesysteme, die auf chemischen und/oder natürlichen Farbstoffen basieren. Die durch permanente, semipermanente oder temporäre Färbesysteme künstlich erzeugten Haarfarben weisen jedoch den Nachteil auf, dass sie sich - beispielsweise während oder nach der Haarreinigung - in unerwünschter Weise verändern können.

Unter "unerwünschter Veränderung" wird das Verblassen oder Ausbluten sowie der Verlust der Farbbrillanz des durch die jeweilige Färbung erzielten Farbtons der Haare verstanden. Umwelteinflüsse und/oder Sonneneinwirkungen können diese Veränderungen noch verstärken.

Es besteht demnach der Bedarf nach kosmetischen Verfahren, mit denen künstlich erzeugte Haarfarben besser stabilisiert werden können.

Haarbehandlungsmittel zum Schutz künstlich erzeugter Haarfarben oder Verfahren zur Stabilisierung künstlich erzeugter Haarfarben sind im Prinzip bekannt. In EP 1676604A1 wird ein Verfahren zur Verbesserung des Farbtons von Haaren beschrieben, bei dem die Haare zunächst mit einem Shampoo gewaschen werden, das neben einem anionischen Tensid und einem speziellen Silikon mindestens ein wasserlösliches Salz - bevorzugt Natriumsulfat - enthält. In einem zweiten Schritt werden die Haare mit einem Konditioniermittel, umfassend einen höheren Alkohol und ein kationisches Tensid im einem bestimmten Gewichtsverhältnis behandelt und anschließend ausgespült.

In WO 2002/32386 wird die Verwendung kurzkettiger Carbonsäuren als Wirkstoff zur Farbstabilisierung der Färbung keratinischer Fasern in kosmetischen Mitteln beschrieben.

Eine Haarbehandlungszusammensetzung für gefärbte Haare wird auch in EP 1118319A1 beschrieben. Diese umfasst eine Mischung aromatischer Lösungsmittel, aromatischer Carbonsäure(salze)n und niederen Alkohole, Poyole oder Polyolether, weist einen pH-Wert im Bereich von 1 bis 6 auf und ist frei von Farbstoffen.

Es besteht jedoch der Verbraucherwunsch nach weniger zeitintensiven Verfahren, mit denen Haarfarben bevorzugt in einem Behandlungsschritt vor einer Veränderung geschützt werden können.

Der vorliegenden Erfindung lag die Aufgabe zugrunde, ein einfach und schnell anzuwendendes kosmetisches Behandlungsverfahren zu entwickeln, mit dem die Haftung von Farbstoffen auf den Haarfasern gestärkt, und damit die Echtheit der künstlich erzeugten Haarfarbe erhalten werden kann. Idealerweise sollten bei dem Verfahren milde kosmetische Zusammensetzungen verwendet werden, die den behandelten Haaren neben dem Farbschutz verbesserte optische und haptische Eigenschaften verleihen und die Haare nicht strapazieren.

Es wurde gefunden, dass ein Haarbehandlungsverfahren unter Verwendung einer aciden kosmetischen Zusammensetzung, welche ein spezielles Salz oder eine spezielle Säure-SalzMischung enthält, dafür hervorragend geeignet ist.

Ein erster Gegenstand der Erfindung ist daher ein Verfahren zur Verringerung und/oder Vermeidung des Ausblutens und/oder Verblassens künstlich erzeugter Haarfarben, umfassend die Folgenden Schritte:
i. Aufbringen einer kosmetischen Zusammensetzung auf die - vorzugsweise nassen - gefärbten Haare,
ii. Einwirken lassen der Zusammensetzung für einen Zeitraum von mindestens 5 Sekunden,
iii. Ausspülen der Zusammensetzung mit Wasser, dadurch gekennzeichnet,
   - dass die kosmetische Zusammensetzung - bezogen auf ihr Gewicht -
      a) 0,05 bis 0,5 mol/kg Calciumlactat oder
      b) 0,01 bis 0,5 mol/kg Milchsäure (b1) und 0,05 bis 0,5 mol/kg Calciumchlorid und/oder Calciumhydroxid (b2) enthält, und
   - dass die kosmetische Zusammensetzung einen pH-Wert im Bereich von 4,0 bis 4,8 aufweist.

Unter geeigneten kosmetischen Zusammensetzungen sind bevorzugt Haarreinigungsmittel wie Shampoos, Haarpflegemittel wie Haarkuren, Spülungen oder Haarpflegesprays sowie Haarstylingmittel wie Haargele, Haarsprays oder Haarwachse zu verstehen.

In einer ersten bevorzugten Ausführungsform umfasst das erfindungsgemäße Verfahren die folgenden Schritte
i. Aufbringen eines Shampoos oder einer Haarspülung auf die nassen gefärbten Haare,
ii. Einwirken lassen des Shampoos oder der Haarspülung für einen Zeitraum von 5 Sekunden bis 3 Minuten,
iii. Ausspülen des Shampoos oder der Haarspülung mit Wasser dadurch gekennzeichnet,
   - dass das Shampoo oder die Haarspülung - bezogen auf sein/ihr Gewicht -
      a) 0,05 bis 0,5 mol/kg Calciumlactat oder
      b) 0,01 bis 0,5 mol/kg Milchsäure (b1) und 0,05 bis 0,5 mol/kg Calciumchlorid und/oder Calciumhydroxid (b2) enthält, und
   - dass das Shampoo oder die Haarspülung einen pH-Wert im Bereich von 4,0 bis 4,8 aufweist.

In einer zweiten bevorzugten Ausführungsform umfasst das erfindungsgemäße Verfahren den Schritt des Aufbringens eines Haar(pflege)sprays, Haargels oder Haarwachses auf die trockenen gefärbten Haare, wobei
- das Haar(pflege)spray, Haargel oder Haarwachs - bezogen auf sein Gewicht -
   a) 0,05 bis 0,5 mol/kg Calciumlactat oder
   b) 0,01 bis 0,5 mol/kg Milchsäure (b1) und 0,05 bis 0,5 mol/kg Calciumchlorid und/oder Calciumhydroxid (b2) enthält, und
- einen pH-Wert im Bereich von 4,0 bis 4,8 aufweist.

Die kosmetischen Zusammensetzungen, die in dem erfindungsgemäßen Verfahren verwendet werden, enthalten - bezogen auf ihr Gewicht - bevorzugt
- 0,04 bis 0,40 mol/kg, mehr bevorzugt 0,06 bis 0,30 mol/kg und insbesondere 0,07 bis 0,20 mol/kg Calciumlactat a) oder
- 0,02 bis 0,40 mol/kg, mehr bevorzugt 0,03 bis 0,30 mol/kg und insbesondere 0,05 bis 0,20 mol/kg Milchsäure (b1) sowie
0,05 bis 0,40 mol/kg, mehr bevorzugt 0,06 bis 0,30 mol/kg und insbesondere 0,07 bis 0,20 mol/kg Calciumchlorid und/oder Calciumhydroxid (b2).

In einer weiteren bevorzugten Ausführungsform des erfindungsgemäßen Verfahrens enthält die kosmetische Zusammensetzung - bezogen auf ihr Gewicht - daher
- 0,06 bis 0,30 mol/kg und insbesondere 0,07 bis 0,20 mol/kg Calciumlactat a) oder
- 0,02 bis 0,40 mol/kg, mehr bevorzugt 0,03 bis 0,30 mol/kg und insbesondere 0,05 bis 0,20 mol/kg Milchsäure (b1) sowie
0,05 bis 0,40 mol/kg, mehr bevorzugt 0,06 bis 0,30 mol/kg und insbesondere 0,07 bis 0,20 mol/kg Calciumchlorid und/oder Calciumhydoxid (b2).

Es wurde gefunden, dass das erfindungsgemäße Verfahren besonders effektiv und die Farbveränderung der künstlich erzeugten Haarfarbe nach mehreren Reinigungsvorgängen besonders gering ist, wenn die kosmetischen Zusammensetzungen Calciumlactat oder eine Mischung aus Milchsäure und Calciumchlorid und/oder Calciumhydroxid enthalten und einen pH-Wert im Bereich von 4,1 bis 4,75, bevorzugt von 4,2 bis 4,7 und insbesondere von 4,3 bis 4,6 aufweisen.

In einer weiteren bevorzugten Ausführungsform des erfindungsgemäßen Verfahrens weist die kosmetische Zusammensetzung daher bevorzugt einen pH-Wert im Bereich von 4,1 bis 4,75, mehr bevorzugt von 4,2 bis 4,7 und insbesondere von 4,3 bis 4,6 auf.

Das erfindungsgemäße Verfahren ist prinzipiell anwendbar auf Haaren, die mit permanenten, semipermanenten oder temporären Haarfarben gefärbt wurden. Temporäre Haarfarben sind jedoch dafür bestimmt, mit der Zeit ausgewaschen zu werden und/oder zu verblassen, deshalb ist das erfindungsgemäße Verfahren besonders geeignet für die Anwendung auf Haaren, die mit permanenten oder oxidativen Haarfärbemitteln gefärbt wurden.

In einer weiteren bevorzugten Ausführungsform wird das erfindungsgemäße Verfahren daher auf Haaren angewendet, die mit oxidativen Haarfärbemitteln gefärbt wurden.

Ein zweiter Gegenstand der Erfindung ist eine kosmetische Reinigungs- oder Pflegezusammensetzung zur Verringerung und/oder Vermeidung des Ausblutens und/oder Verblassens künstlich erzeugter Haarfarben, die - bezogen auf ihr Gewicht -
a)
   (i) 0,05 bis 0,5 mol/kg Calciumlactat oder
   (ii) 0,01 bis 0,5 mol/kg Milchsäure (b1) und 0,05 bis 0,5 mol/kg Calciumchlorid und/oder Calciumhydoxid (b2), und
   b) mindestens ein Tensid, ausgewählt aus
   - anionischen Tensiden der Gruppe, die gebildet wird aus Alkyl(ether)sulfaten, Sulfosuccinaten, Ethercarbonsäuren, Acylglutamat- und/oder Acylisethionaten mit jeweils 8 bis 24 C-Atomen in der Acylgruppe sowie Mischungen dieser Tenside enthält,
   und einen pH-Wert im Bereich von 4,2 bis 4,7 aufweist.

Unter kosmetischen Reinigungs- und Pflegezusammensetzungen werden bevorzugt Haarshampoos, Haarspülungen und/oder Haarkuren verstanden, die neben den zuvor genannten Inhaltsstoffen weitere, in den jeweiligen Mittel üblichen, Inhaltsstoffe enthalten können.

Weitere übliche Inhaltsstoffe und Beispiele für Standard-Formulierungen der zuvor genannten Haarbehandlungsmittel finden sich beispielsweise in der Monographie Karlheinz Schrader: "Grundlagen und Rezepturen der Kosmetika", 2. Auflage (1989), Hüthig Buch Verlag GmbH Heidelberg, Seiten 676-848.

Haarfarben können sich in der Regel am meisten während oder nach der Haarreinigung verändern. Der Kontakt der Haare mit Wasser und Tensiden, aber auch das Einmassieren des Shampoos, das Trockenrubbeln nach dem Ausspülen des Shampoos oder die Fönhitze beim anschließenden Trocknungsvorgang können die Haftung der Haarfarbe beeinträchtigen und zu einer unerwünschten Farbveränderung und/oder zu weniger Brillanz der künstlich erzeugten Haarfarbe führen. Es ist daher besonders erstrebenswert, dass der Reinigungsvorgang gefärbter Haare besonders schonend erfolgen kann.

Es wurde gefunden, dass der Reinigungsvorgang gefärbter Haare besonders schonend gelingt, wenn die Reinigungs- und Pflegezusammensetzung des zweiten Erfindungsgegenstandes als Haarshampoo oder als Pflegespülung konfektioniert wird, die direkt am Anschluss an die Haarreinigung angewendet wird.

In einer ersten bevorzugten Ausführungsform des zweiten Erfindungsgegenstandes wird die erfindungsgemäße Reinigungs- und Pflegezusammensetzung daher als Haarshampoo konfektioniert und enthält bevorzugt mindestens ein anionisches Tensid, in einem bevorzugten Gewichtsanteil von 0,5 bis 20 Gew.-%, mehr bevorzugt von 1 bis 15 und insbesondere von 2 bis 12 Gew.-%, wobei sich die Mengenangaben auf das Gesamtgewicht der Reinigungs- oder Pflegezusammensetzung beziehen.

Zu der Gruppe der besonders bevorzugten anionischen Tenside zählen beispielsweise:
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylglutamat- und/oder Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, und/oder
- Alkylsulfat- und/oder Alkylpolyglykolethersulfatsalze der Formel R-(OCH₂-CH₂)ₓ-OSO₃⁻X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet.

Besonders bevorzugte anionische Tenside sind geradkettige oder verzweigte Alkylethersulfate der zuvor genannten Formel, die einen Alkylrest mit 8 bis 18, insbesondere mit 10 bis 16 C-Atomen, sowie 1 bis 6 und insbesondere 2 bis 4 Ethylenoxideinheiten enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

In einer weiteren bevorzugten Ausführungsform des zweiten Erfindungsgegenstandes enthält die kosmetische Reinigungs- und/oder Pflegezusammensetzung - bezogen auf ihr Gewicht
- 0,06 bis 0,30 mol/kg und insbesondere 0,07 bis 0,20 mol/kg Calciumlactat a) oder
- bevorzugt 0,02 bis 0,40 mol/kg, mehr bevorzugt 0,03 bis 0,30 mol/kg und insbesondere 0,05 bis 0,20 mol/kg Milchsäure (b1) sowie bevorzugt 0,05 bis 0,40 mol/kg, mehr bevorzugt 0,06 bis 0,30 mol/kg und insbesondere 0,07 bis 0,20 mol/kg Calciumchlorid und/oder Calciumhydroxid (b2).

Es wurde gefunden, dass die Wirksamkeit der Reinigungs- und/oder Pflegezusammensetzung noch weiter gesteigert werden kann, wenn Ihr ein spezieller haarkonditionierender Wirkstoff hinzugefügt wird. Insbesondere konnte dadurch die Farbbrillanz der künstlich erzeugten Haarfarbe stabilisiert und erhalten werden.

In einer weiteren bevorzugten Ausführungsform enthält die Reinigungs- und/oder Pflegezusammensetzung des zweiten Erfindungsgegenstandes daher zusätzlich mindestens einen haarkonditionierenden Wirkstoff in einem Gewichtsanteil von 0,01 bis 10 Gew.-% am Gesamtgewicht der kosmetischen Reinigungs- oder Pflegezusammensetzung.

Unter geeigneten haarkonditionierenden Wirkstoffen werden bevorzugt kationische Pflegepolymere, natürliche, mineralische oder synthetische Öl-, Fett- oder Wachskomponenten, Vitamine und/oder Proteinhydrolysate verstanden.

Unter geeigneten Proteinhydrolysaten sind Produktgemische zu verstehen, die durch sauer, basisch oder enzymatisch katalysierten Abbau von Proteinen (Eiweißen) erhalten werden können.

Es können Proteinhydrolysate pflanzlichen, tierischen und/oder marinen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Das oder die Proteinhydrolysate kann (können) in der Reinigungs- oder Pflegezusammensetzung des zweiten Erfindungsgegenstandes - bezogen auf deren Gesamtgewicht - bevorzugt in Mengen von 0,01 bis 5 Gew.-%, mehr bevorzugt von 0,025 bis 3 Gew.-% und insbesondere von 0,05 bis 2 Gew.-% eingesetzt werden.

Geeignete kationische Polymere sind beispielsweise:
- quaternisierte Cellulose-Derivate, wie sie unter den Bezeichnungen Celquat^{®} und Polymer JR^{®} im Handel erhältlich sind,
- hydrophob modifizierte Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cosmedia^{®}Guar N-Hance^{®} und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol, sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Bevorzugte kationische Polymere sind kationische Polysaccharidpolymere wie quaternisierte Cellulosepolymere, hydrophob modifizierte kationische Cellulosederivate und/oder kationische Guarderivate die besonders bevorzugt ausgewählt sind aus den unter den INCI-Bezeichnungen bekannten Polymeren Guar Hydroxypropyltrimonium Chloride, Polyquaternium-10 und/oder Polyquaternium-67.

Das oder die kationische(n) Polymer(e), kann (können) in der Reinigungs- oder Pflegezusammensetzung des zweiten Erfindungsgegenstandes - bezogen auf deren Gesamtgewicht - bevorzugt in einer Menge 0,01 bis 10 Gew.-%, mehr bevorzugt 0,05 bis 5 Gew.-% und insbesondere 0,1 bis 3 Gew.-% eingesetzt werden.

Unter geeigneten Vitaminen sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   ➢ Vitamin B₁ (Thiamin)
   ➢ Vitamin B₂ (Riboflavin)
   ➢ Vitamin B₃. Unter dieser Bezeichnung werden häufig die Verbindungen Nicotinsäure und Nicotinsäureamid (Niacinamid) geführt.
   ➢ Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   ➢ Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Die erfindungsgemäße Reinigungs- oder Pflegezusammensetzung des zweiten Erfindungsgegenstandes kann bevorzugt Vitamine, Provitamine und Vitaminvorstufen aus den Gruppen A, B, E und H enthalten.

Insbesondere bevorzugt sind Nicotinsäureamid, Biotin, Pantolacton und/oder Panthenol.

Vitamine, Vitaminderivate und/oder Vitaminvorstufen können in der erfindungsgemäßen Reinigungs- oder Pflegezusammensetzung des zweiten Erfindungsgegenstandes - bezogen auf deren Gesamtgewicht - bevorzugt in einer Menge von 0,001 bis 2 Gew.-%, besonders bevorzugt von 0,005 bis 1 Gew.-% und insbesondere von 0,01 bis 0,5 Gew.-% eingesetzt werden.

Unter geeigneten natürlichen (pflanzlichen) Ölen werden üblicherweise Triglyceride und Mischungen von Triglyceriden verstanden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und/oder Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).

Als Ölkomponente kann weiterhin ein Dialkylether dienen.

Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Besonders bevorzugt ist der Di-n-octylether, der im Handel unter der Bezeichnung Cetiol^{®} OE erhältlich ist.

Als synthetische Öle kommen bevorzugt Silikonverbindungen in Betracht.

Silikone bewirken auf dem Haar ausgezeichnete konditionierende Eigenschaften. Insbesondere bewirken sie eine bessere Kämmbarkeit der Haare in nassem und trockenem Zustand und wirken sich in vielen Fällen positiv auf den Haargriff und die Weichheit der Haare aus.

Es ist daher erstrebenswert, in kosmetischen Haarbehandlungsmitteln Silikone einzusetzen. Geeignete Silikone können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Unter Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

### Weitere Fettstoffe sind beispielsweise

- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-O18, Monomuls^{®} 90-L12, Cetiol^{®} HE oder Cutina^{®} MD.

Der Gewichtsanteil der Öl-, Wachs- und/oder Fettkomponenten am Gesamtgewicht der erfindungsgemäßen Reinigungs- oder Pflegezusammensetzung des zweiten Erfindungsgegenstandes beträgt bevorzugt 0,01 bis 10 Gew.-%, besonders bevorzugt 0,025 bis 7,5 Gew.-% und insbesondere 0,05 bis 5 Gew.-%.

Es wurde gefunden, dass die Farbbrillanz der Haare mit den erfindungsgemäßen Reinigungs- oder Pflegezusammensetzungen des zweiten Erfindungsgegenstandes besonders gut stabilisiert werden kann, wenn die Reinigungs- oder Pflegezusammensetzungen als haarkonditionierenden Wirkstoff mindestens ein kationisches Polymer, bevorzugt ein kationisches Polysaccharid, und/oder mindestens ein pflanzliches Öl und/oder ein Silikon enthalten. Durch die Zugabe dieser speziellen Pflegestoffe konnten außerdem die haptischen Eigenschaften wie der Griff und die Geschmeidigkeit der gefärbten Haare verbessert werden.

In einer weiteren bevorzugten Ausführungsform enthalten die Reinigungs- und/oder Pflegezusammensetzungen des zweiten Erfindungsgegenstandes daher zusätzlich als haarkonditionierenden Wirkstoff ein kationisches Polymer - insbesondere ein kationisches Polysaccharid - und/oder ein pflanzliches Öl und/oder ein Silikon.

Chelatisierungs- und/oder Komplexierungsmittel, insbesondere Stickstoff-enthaltende Komplexierungs- und/oder Chelatisierungsmittel, können sich negativ auf die zwingend in den Reinigungs- oder Pflegezusammensetzungen enthaltenden Salze auswirken, indem sie die Kationen der teilweise bis vollständig gelösten Salze komplexieren und damit ihre Wirksamkeit stören.

In einer weiteren bevorzugten Ausführungsform sind die Reinigungs- oder Pflegezusammensetzungen des zweiten Erfindungsgegenstandes daher im Wesentlichen frei von Stickstoff-enthaltenden Komplexierungs- und/oder Chelatisierungsmitteln.

Unter "im Wesentlichen frei" wird verstanden, dass die Reinigungs- oder Pflegezusammensetzungen maximal 0,01 Gew.-%, bevorzugt maximal 0,005 und insbesondere bevorzugt maximal 0,001 Gew.-% Stickstoff-enthaltende Komplexierungs- und/oder Chelatisierungsmittel enthalten.

In einer weiteren bevorzugten Ausführungsform sind erfindungsgemäße Reinigungs- oder Pflegezusammensetzungen des zweiten Erfindungsgegenstandes dadurch gekennzeichnet, dass sie - bezogen auf ihr Gewicht -
a) bevorzugt 0,04 bis 0,40 mol/kg, mehr bevorzugt 0,06 bis 0,30 mol/kg und insbesondere 0,07 bis 0,20 mol/kg Calciumlactat oder bevorzugt 0,02 bis 0,40 mol/kg, mehr bevorzugt 0,03 bis 0,30 mol/kg und insbesondere 0,05 bis 0,20 mol/kg Milchsäure sowie bevorzugt 0,05 bis 0,40 mol/kg, mehr bevorzugt 0,06 bis 0,30 mol/kg und insbesondere 0,07 bis 0,20 mol/kg Calciumchlorid oder Calciumhydroxid,
b) bevorzugt 0,5 bis 20 Gew.-%, mehr bevorzugt von 1 bis 15 und insbesondere von 2 bis 12 Gew.-% mindestens eines anionischen Tensids und
c) bevorzugt 0,01 bis 10 Gew.-%, mehr bevorzugt 0,05 bis 5 Gew.-% und insbesondere 0,1 bis 3 Gew.-% mindestens eines kationischen Polysaccharids, enthalten, und einen pH-Wert im Bereich von 4,2 bis 4,7, bevorzugt von 4,25 bis 4,65 und insbesondere von 4,3 bis 4,6 aufweisen.

Innerhalb dieser Ausführungsform sind Reinigungs- oder Pflegezusammensetzungen des zweiten Erfindungsgegenstandes besonders bevorzugt, die
a) bevorzugt 0,04 bis 0,40 mol/kg, mehr bevorzugt 0,06 bis 0,30 mol/kg und insbesondere 0,07 bis 0,20 mol/kg Calciumlactat oder bevorzugt 0,02 bis 0,40 mol/kg, mehr bevorzugt 0,03 bis 0,30 mol/kg und insbesondere 0,05 bis 0,20 mol/kg Milchsäure sowie bevorzugt 0,05 bis 0,40 mol/kg, mehr bevorzugt 0,06 bis 0,30 mol/kg und insbesondere 0,07 bis 0,20 mol/kg Calciumchlorid oder Calciumhydroxid,
b) bevorzugt 0,5 bis 20 Gew.-% mindestens eines Alkylsulfat- und/oder Alkylpolyglykolethersulfatsalzes der Formel R-(OCH₂-CH₂)ₓ-OSO₃⁻X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet, und
c) bevorzugt 0,01 bis 10 Gew.-%, mindestens eines der unter den INCI-Bezeichnungen Polyquaternium-10, Guar Hydroxypropyltrimonium Chloride und/oder Polyquaternium-67 bekannten kationischen Polysaccharide, enthalten, und einen pH-Wert im Bereich von 4,3 bis 4,6 aufweisen.

In einer weiteren bevorzugten Ausführungsform sind erfindungsgemäße Reinigungs- oder Pflegezusammensetzungen des zweiten Erfindungsgegenstandes dadurch gekennzeichnet, dass sie - bezogen auf ihr Gewicht -
a) bevorzugt 0,04 bis 0,40 mol/kg, mehr bevorzugt 0,06 bis 0,30 mol/kg und insbesondere 0,07 bis 0,20 mol/kg Calciumlactat oder
   bevorzugt 0,02 bis 0,40 mol/kg, mehr bevorzugt 0,03 bis 0,30 mol/kg und insbesondere 0,05 bis 0,20 mol/kg Milchsäure sowie bevorzugt 0,05 bis 0,40 mol/kg, mehr bevorzugt 0,06 bis 0,30 mol/kg und insbesondere 0,07 bis 0,20 mol/kg Calciumchlorid oder Calciumhydroxid,
b) bevorzugt 0,5 bis 20 Gew.-%, mehr bevorzugt von 1 bis 15 und insbesondere von 2 bis 12 Gew.-% mindestens eines anionischen Tensids und/oder bevorzugt 0,01 bis 5 Gew.-%, mehr bevorzugt von 0,05 bis 2 Gew.-% und insbesondere von 0,1 bis 0,5 Gew.-% mindestens eines kationischen Tensids, und
c) bevorzugt 0,01 bis 10 Gew.-%, besonders bevorzugt 0,025 bis 7,5 Gew.-% und insbesondere 0,05 bis 5 Gew.-% mindestens einer natürlichen, mineralischen oder synthetischen Öl-, Fett- oder Wachskomponente enthalten, und einen pH-Wert im Bereich von 4,2 bis 4,7, bevorzugt von 4,25 bis 4,65 und insbesondere von 4,3 bis 4,6 aufweisen.

Innerhalb dieser Ausführungsform sind Reinigungs- oder Pflegezusammensetzungen des zweiten Erfindungsgegenstandes besonders bevorzugt, die
a) bevorzugt 0,04 bis 0,40 mol/kg, mehr bevorzugt 0,06 bis 0,30 mol/kg und insbesondere 0,07 bis 0,20 mol/kg Calciumlactat oder
   bevorzugt 0,02 bis 0,40 mol/kg, mehr bevorzugt 0,03 bis 0,30 mol/kg und insbesondere 0,05 bis 0,20 mol/kg Milchsäure sowie bevorzugt 0,05 bis 0,40 mol/kg, mehr bevorzugt 0,06 bis 0,30 mol/kg und insbesondere 0,07 bis 0,20 mol/kg Calciumchlorid oder Calciumhydroxid,
b) bevorzugt 0,5 bis 20 Gew.-% mindestens eines Alkylsulfat- und/oder Alkylpolyglykolethersulfatsalzes der Formel R-(OCH₂-CH₂)ₓ-OSO₃⁻X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet, und
d) bevorzugt 0,01 bis 10 Gew.-% mindestens eines pflanzlichen Öls und/oder eines Silikons enthalten, und
   einen pH-Wert im Bereich von 4,3 bis 4,6 aufweisen.

Die Reinigungs- oder Pflegezusammensetzungen des zweiten Erfindungsgegenstandes können weitere bevorzugte Inhaltsstoffe umfassen, die ihnen vorteilhafte Eigenschaften verleihen, darunter
- nichtionische und/oder amphotere Tenside,
- Antischuppenwirkstoffe,
- Perlglanzmittel und/oder Trübungsmittel.

Zu der Gruppe der geeigneten nichtionischen Tenside zählen beispielsweise:
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Fettsäurealkanolamide,
- Zuckerfettsäureester und Anlagerungsprodukte von Ethylenoxid an Zuckerfettsäureester,
- Anlagerungsprodukte von Ethylenoxid an Fettsäurealkanolamide und Fettamine und/oder
- Alkylpolyglucoside der allgemeinen Formel RO-[G]ₓ, in der R für einen Alkyl- und/oder Alkenylrest mit 4 bis 22 C-Atomen, G für einen Zuckerrest mit 5 oder 6 C-Atomen und x für Zahlen von 1 bis 10 steht.

Besonders geeignete Alkylpolyglucoside leiten sich von Aldosen und/oder Ketosen mit 5 oder 6 Kohlenstoffatomen, vorzugsweise von Glucose ab. Der Rest R steht besonders bevorzugt für einen Alkylrest mit 6 bis 20 und insbesondere mit 8 bis 18 Kohlenstoffatomen. Die Indexzahl x steht in der allgemeinen Formel RO-[G][x] für den Oligomerisierungsgrad (DP), d. h. für die Verteilung der Mono- und Oligoglycoside. Die Indexzahl x weist vorzugsweise einen Wert im Bereich von 1 bis 6, besonders bevorzugt im Bereich von 1 bis 3 auf, wobei es sich dabei um keine ganze Zahl, sondern um eine gebrochene Zahl handeln kann, die analytisch ermittelt werden kann. Insbesondere bevorzugte Alkylpolyglucoside weisen einen Oligomerisierungsgrad zwischen 1,2 und 1,5 auf. Insbesondere geeignete Alkylpolyglucoside sind bekannt und im Handel von verschiedenen Anbietern erhältlich unter den INCI-Bezeichnungen Decyl Glucoside, Lauryl Glucoside und Coco Glucoside.

Besonders geeignete nichtionische Tenside/Emulgatoren sind Alkylpolyglucoside, insbesondere Alkylpolyglucoside auf der Basis von gehärtetem C₁₀₋₁₄-Kokosalkohol mit einem DP von 1-3, wie sie beispielsweise unter der INCI-Bezeichnung "Coco-Glucoside" im Handel erhältlich sind.

Das (oder die) nichtionische(n) Tensid(e) wird (werden) in den Reinigungs- oder Pflegezusammensetzungen des zweiten Erfindungsgegenstandes - bezogen auf deren Gesamtgewicht - bevorzugt in einer Menge von 1 bis 15 Gew.-%, mehr bevorzugt von 1,5 bis 14 Gew.-%, besonders bevorzugt von 2 bis 12,5 Gew.-% und insbesondere von 2,5 bis 10 Gew.-% eingesetzt.

Zu der Gruppe der geeigneten amphoteren und/oder zwitterionischen Tenside zählen die sogenannten Betaine wie die N-Alkyl-N,N-dimethylammonium-glycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Unter amphoteren Tensiden werden solche oberflächenaktiven Verbindungen verstanden, die zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Aminopropionate, Aminoglycinate, Imidazoliniumbetaine und Sulfobetaine.

Besonders bevorzugte amphotere Tenside sind das N-Kokosalkylaminopropionat, das Kokosacylaminoethylaminopropionat, das unter der INCI-Bezeichnung Cocamidopropyl Betaine bekannte Fettsäureamid-Derivat und das C₁₂ - C₁₈ - Acylsarcosin.

Die Gesamtmenge an amphoteren und/oder zwitterionischen Tensiden in den Reinigungs- oder Pflegezusammensetzungen des zweiten Erfindungsgegenstandes beträgt bevorzugt 1 bis 6 Gew.%, mehr bevorzugt von 1,2 bis 5 Gew.%, besonders bevorzugt 1,4 bis 4,5 Gew.% und insbesondere 1,5 bis 4 Gew.%, wobei sich die Mengenangaben auf das Gesamtgewicht der kosmetischen Zusammensetzungen beziehen.

Antischuppenwirkstoffe können in den Reinigungs- oder Pflegezusammensetzungen des zweiten Erfindungsgegenstandes - bezogen auf deren Gesamtgewicht - bevorzugt in Mengen von 0,01 bis 10 Gew.-%, mehr bevorzugt von 0,025 bis 7,5 Gew.-%, besonders bevorzugt von 0,05 bis 5 Gew.-% und insbesondere von 0,075 bis 3 Gew.-% eingesetzt werden.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten. Bevorzugt sind Climbazol, Zink Pyrithion, Piroctone Olamine und/oder Salicylsäure.

Geeignete Trübungs- und/oder Perlglanzmittel können in den Reinigungs- oder Pflegezusammensetzungen des zweiten Erfindungsgegenstandes - bezogen auf deren Gesamtgewicht - bevorzugt in Mengen von (jeweils) 0,001 bis 5 Gew.-%, mehr bevorzugt von 0,005 bis 4 Gew.-%, besonders bevorzugt von 0,01 bis 3 Gew.-% und insbesondere von 0,05 bis 2 Gew.-% eingesetzt werden.

Unter geeigneten Perlglanzmitteln und Trübungsmitteln sind beispielsweise
- Mono- und/oder Diester des Ethylenglycols, 1,2-Propandiols und/oder Glycerins mit C₈-C₂₄-Fettsäuren,
- Ester aus Polyethylenglycolen mit C₈-C₂₄-Fettsäuren,
- TiO₂ oder TiO₂-beschichtetes synthetisches oder natürliches Mica und/oder
- Styrol/Acrylat-Copolymere zu verstehen.

Besonders geeignet sind die unter den INCI-Bezeichnungen bekannten Trübungs- und/oder Perlglanzmittel:
Glycol Distearate, wie beispielsweise das Handelsprodukt Cutina^{®} AGS der Firma Cognis, Glycol Monostearate, wie beispielsweise das Handelsprodukt Cutina^{®} EGMS der Firma Cognis, PEG-3 Distearate, wie beispielsweise das Handelsprodukt Genapol^{®} TS der Firma Clariant, PEG-2 Distearate, wie beispielsweise das Handelsprodukt Kessco^{®} DEGMS der Firma Akzo Nobel, Propylen Glycol Stearate, wie beispielsweise das Handelsprodukt Tegin^{®} P der Firma Goldschmidt und/oder Styrol/Acrylates-Copolymere wie beispielsweise die Handelsprodukte Joncryl^{®} 67 der Firma Johnson Polymers, Suprawal^{®} WS der Firma BASF und/oder Acusol^{®} OP 301 der Firma Rohm & Haas.

Weitere Wirk-, Hilfs- und Zusatzstoffe, die in den erfindungsgemäßen Reinigungs- oder Pflegezusammensetzungen des zweiten Erfindungsgegenstandes enthalten können, sind beispielsweise:
- Pflanzenextrakte,
- Feuchthaltemittel,
- Parfums,
- UV-Filter,
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn- Seifen,
- Strukturanten wie Maleinsäure und Milchsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Wirkstoffe wie Bisabolol,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konservierungsmittel, wie beispielsweise Natriumbenzoat oder Salicylsäure,
- Viskositätsregler wie Salze (NaCl).

Ein dritter Gegenstand der Erfindung ist die Verwendung der erfindungsgemäßen Reinigungs- oder Pflegezusammensetzungen zur Verringerung und/oder Vermeidung des Ausblutens und/oder Verblassens künstlich erzeugter Haarfarben.

### Beispiele:

### 1) Ausführunqsbeispiele: Es wurden die folgenden erfindungsgemäßen Reinigungs- und Pflegezusammensetzungen hergestellt (die Mengenangaben beziehen sich dabei auf Gew.-%):

**Farbschutz-Shampoos**

| | **Shampoo** 1 | **Shampoo** 2 |
|---|---|---|
| Sodium Laureth Sulfate | 11,00 | 11,00 |
| Cocamidopropyl Betaine | 1,00 | 1,50 |
| Disodium Cocoamphodiacetate | 0,50 | |
| Cocamide MEA | 0,50 | 0,50 |
| PEG-12 Dimethicone | 0,50 | 0,30 |
| Glycol Distearate | 1,20 | |
| PEG-7 Glyceryl Cocoate | 0,40 | 0,60 |
| Polyquaternium-10 | 0,90 | 0,60 |
| Panthenol | 0,30 | 0,20 |
| Aprikosenkernöl | 0,05 | |
| Hydrogenated Castor Oil | 0,20 | 0,10 |
| Lactic Acid | 2,23 | |
| Calcium Hydroxide | 0,542 | |
| Calciumlactate | | 1,50 |
| Konservierungsmittel, Parfum, evtl. Säuerungsmittel | q.s. | q.s. |
| Wasser | ad 100 | ad 100 |
| pH | 4,5 | 4,4 |

Das o.g. Shampoo 1 wurde gemäß dem erfindungsgemäßen Verfahren auf die nassen Haare aufgetragen und nach einer Einwirkungszeit von etwa einer Minute mit Wasser ausgespült. Die Haare wiesen nach der Reinigung eine brillante Haarfarbe auf, die sich nicht verändert hatte. Zudem wiesen sie einen optisch ansprechenden Glanz auf und fühlten sich weich an.

### 2) Wirkunasnachweise: Es wurden Haarsträhnen untersucht, die jeweils mit gleichen Mengen der gleichen handelsüblichen, permanenten Haarfärbezusammensetzung gefärbt wurden.

Eine Haarsträhne wurde nach dem erfindungsgemäßen Verfahren mit einem Pflegeshampoo behandelt, das 0,3 mol/kg Calciumlactat enthielt, während weitere 5 Haarsträhnen jeweils mit gleichen Mengen im Handel erhältlicher Farbschutzshampoos, die kein Calciumlactat enthalten, in der gleichen Weise gewaschen wurden. Die Behandlungsvorgänge wurden je Haarsträhne 5 Mal wiederholt. Anschließend wurden jeweils die Delta-E-Werte bestimmt (s.u.). Die Ergebnisse lassen erkennen, dass sich die Haarfarbe nach der Behandlung mit dem Pflegeshampoo 2. am wenigsten verändert.

**Die folgenden Shampoos wurden untersucht:**

| Shampoos | pH-Wert |
|---|---|
| 1. Pflegeshampoo ohne Calciumlactat | 4,50 |
| 2. Pflegeshampoo mit 0,3 Gew.-% Calciumlactat | 4,50 |
| 3. Farbschutzshampoo Marktprodukt 1 | 5,70 |
| 4. Farbschutzshampoo Marktprodukt 2 | 5,47 |
| 5. Farbschutzshampoo Marktprodukt 3 | 4,32 |
| 6. Farbschutzshampoo Marktprodukt 4 | 5,46 |

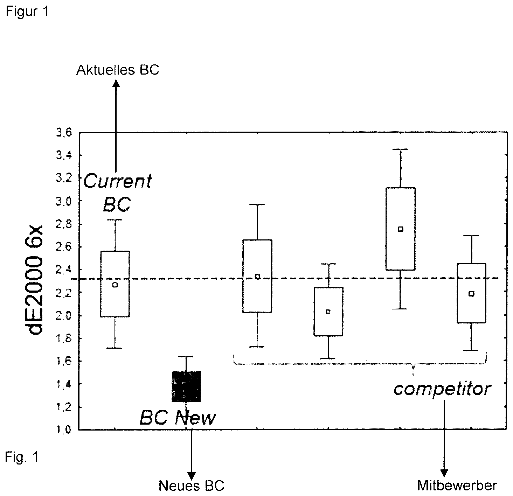

Die nachfolgende Tabelle gibt die ermittelten Delta-E-Werte wieder (vgl. hierzu Fig. 1).

## Patentansprüche

1. Verfahren zur Verringerung und/oder Vermeidung des Ausblutens und/oder Verblassens künstlich erzeugter Haarfarben, umfassend die folgenden Schritte:
i. Aufbringen einer kosmetischen Zusammensetzung auf die - vorzugsweise nassen - gefärbten Haare,
ii. Einwirken lassen der Zusammensetzung für einen Zeitraum von mindestens 5 Sekunden,
iii. Ausspülen der Zusammensetzung mit Wasser, **dadurch gekennzeichnet,**
- **dass** die kosmetische Zusammensetzung - bezogen auf ihr Gewicht -
a) 0,05 bis 0,5 mol/kg Calciumlactat oder
b) 0,01 bis 0,5 mol/kg Milchsäure (b1) und 0,05 bis 0,5 mol/kg Calciumchlorid und/oder Calciumhydroxid (b2) enthält, und
- **dass** die kosmetische Zusammensetzung einen pH-Wert im Bereich von 4,0 bis 4,8 aufweist.

2. Verfahren nach Anspruch 1, **dadurch gekennzeichnet, dass** die kosmetische Zusammensetzung - bezogen auf ihr Gewicht -
- 0,06 bis 0,30 mol/kg und insbesondere 0,07 bis 0,20 mol/kg Calciumlactat oder
- 0,02 bis 0,40 mol/kg, bevorzugt 0,03 bis 0,30 mol/kg und insbesondere 0,05 bis 0,20 mol/kg Milchsäure (b1) sowie 0,05 bis 0,40 mol/kg, bevorzugt 0,06 bis 0,30 mol/kg und insbesondere 0,07 bis 0,20 mol/kg Calciumchlorid und/oder Calciumhydroxid (b2) enthält.

3. Verfahren nach einem der vorhergehenden Ansprüche, **dadurch gekennzeichnet, dass** der pH-Wert der kosmetischen Zusammensetzung im Bereich von 4,1 bis 4,75, bevorzugt von 4,2 bis 4,7 und insbesondere von 4,3 bis 4,6 liegt.

4. Verfahren nach einem der vorhergehenden Ansprüche, **dadurch gekennzeichnet, dass** die Haare mit oxidativen Haarfärbemitteln gefärbt wurden.

5. Kosmetische Reinigungs- oder Pflegezusammensetzung zur Verringerung und/oder Vermeidung des Ausblutens und/oder Verblassens künstlich erzeugter Haarfarben, enthaltend
- bezogen auf ihr Gewicht -
a)
(i) 0,05 bis 0,5 mol/kg Calciumlactat oder
(ii) 0,01 bis 0,5 mol/kg Milchsäure (b1) und 0,05 bis 0,5 mol/kg Calciumchlorid und/oder Calciumhydroxid (b2)
b) mindestens ein Tensid, ausgewählt aus anionischen Tensiden der Gruppe, die gebildet wird aus Alkyl(ether)sulfaten, Sulfosuccinaten, Ethercarbonsäuren, Acylglutamat- und/oder Acylisethionaten mit jeweils 8 bis 24 C-Atomen in der
Acylgruppe sowie Mischungen dieser Tenside, und/oder **dadurch gekennzeichnet, dass** die Reinigungs- oder Pflegezusammensetzung einen pH-Wert im Bereich von 4,2 bis 4,7 aufweist.

6. Kosmetische Reinigungs- oder Pflegezusammensetzung nach Anspruch 5, **dadurch gekennzeichnet, dass** sie mindestens ein anionisches Tensid in einem Gewichtsanteil von 0,5 bis 20 Gew.-%, bevorzugt von 1 bis 15 und insbesondere von 2 bis 12 Gew.-% enthält, wobei sich die Mengenangaben auf das Gesamtgewicht der Reinigungs- oder Pflegezusammensetzung beziehen.

7. Kosmetische Reinigungs- oder Pflegezusammensetzung nach einem der Ansprüche 5 oder 6, **dadurch gekennzeichnet, dass** sie - bezogen auf ihr Gewicht -
- 0,06 bis 0,30 mol/kg und insbesondere 0,07 bis 0,20 mol/kg Calciumlactat oder
- 0,02 bis 0,40 mol/kg, bevorzugt 0,03 bis 0,30 mol/kg und insbesondere 0,05 bis 0,20 mol/kg Milchsäure (b1) sowie 0,05 bis 0,40 mol/kg, bevorzugt 0,06 bis 0,30 mol/kg und insbesondere 0,07 bis 0,20 mol/kg Calciumchlorid und/oder Calciumhydroxid (b2) enthält.

8. Kosmetische Reinigungs- oder Pflegezusammensetzung nach einem der Ansprüche 5 bis 7, **dadurch gekennzeichnet, dass** sie zusätzlich mindestens einen haarkonditionierenden Wirkstoff in einem Gewichtsanteil von 0,01 bis 10 Gew.-% am Gesamtgewicht der kosmetischen Reinigungs- oder Pflegezusammensetzung enthält.

9. Kosmetische Reinigungs- oder Pflegezusammensetzung nach Anspruch 8, **dadurch gekennzeichnet, dass** der mindestens eine haarkonditionierende Wirkstoff ausgewählt ist aus kationischen Polymeren, insbesondere aus kationischen Polysacchariden, und/oder aus pflanzlichen Ölen und/oder Silikonen.

10. Kosmetische Reinigungs- oder Pflegezusammensetzung nach einem der Ansprüche 5 bis 9, **dadurch gekennzeichnet, dass** sie im Wesentlichen frei von Stickstoff-enthaltenden Komplexierungs- und/oder Chelatisierungsmitteln ist.

11. Kosmetische Reinigungs- oder Pflegezusammensetzung nach einem der Ansprüche 5 bis 10, enthaltend - bezogen auf ihr Gewicht -
a) 0,04 bis 0,40 mol/kg, bevorzugt 0,06 bis 0,30 mol/kg und insbesondere 0,07 bis 0,20 mol/kg Calciumlactat oder
0,02 bis 0,40 mol/kg, bevorzugt 0,03 bis 0,30 mol/kg und insbesondere 0,05 bis 0,20 mol/kg Milchsäure sowie 0,05 bis 0,40 mol/kg, bevorzugt 0,06 bis 0,30 mol/kg und insbesondere 0,07 bis 0,20 mol/kg Calciumchlorid oder Calciumhydroxid,
b) 0,5 bis 20 Gew.-%, bevorzugt von 1 bis 15 und insbesondere von 2 bis 12 Gew.-% mindestens eines anionischen Tensids und/oder 0,01 bis 5 Gew.-%, bevorzugt von 0,05 bis 2 Gew.-% und insbesondere von 0,1 bis 0,5 Gew.-% mindestens eines kationischen Tensids, und
c) 0,01 bis 10 Gew.-%, bevorzugt 0,05 bis 5 Gew.-% und insbesondere 0,1 bis 3 Gew.-% mindestens eines kationischen Polysaccharids,
**dadurch gekennzeichnet, dass** die Zusammensetzung einen pH-Wert im Bereich von 4,2 bis 4,7, bevorzugt von 4,25 bis 4,65 und insbesondere von 4,3 bis 4,6 aufweist.

12. Verwendung einer kosmetischen Zusammensetzung nach einem der Ansprüche 5 bis 11 zur Verringerung und/oder Vermeidung des Ausblutens und/oder Verblassens künstlich erzeugter Haarfarben.

## Claims

1. A method for reducing and/or preventing bleeding and/or fading of artificially produced hair colors, comprising the following steps:
i. application of a cosmetic composition to the - preferably wet - dyed hair,
ii. allow the composition to act for a period of at least 5 seconds,
iii. rinse the composition with water,
**characterized in that**
- the cosmetic composition comprises - by weight -
a) 0,05 to 0,5 mol/kg calcium lactate or
b) 0.01 to 0.5 mol/kg of lactic acid (b1) and 0.05 to 0.5 mol/kg of calcium chloride and/or calcium hydroxide (b2), and
- the cosmetic composition has a pH in the range of 4.0 to 4.8.

2. Process according to claim 1, **characterized in that** the cosmetic composition comprises, by weight
- 0.06 to 0.30 mol/kg and in particular 0.07 to 0.20 mol/kg of calcium lactate or
- 0.02 to 0.40 mol/kg, preferably 0.03 to 0.30 mol/kg and in particular 0.05 to 0.20 mol/kg of lactic acid (b1) and 0.05 to 0.40 mol/kg, preferably 0.06 to 0.30 mol/kg and in particular 0.07 to 0.20 mol/kg of calcium chloride and/or calcium hydroxide (b2).

3. Process according to any one of the preceding claims, **characterized in that** the pH of the cosmetic composition is between 4.1 and 4.75, preferably between 4.2 and 4.7 and in particular between 4.3 and 4.6.

4. Process according to any one of the preceding claims, **characterized in that** the hair has been dyed with oxidative hair dyes.

5. Cosmetic cleansing or conditioning composition for reducing and/or preventing the bleeding and/or fading of artificially produced hair dyes, comprising, by weight
a)
(i) 0,05 to 0,5 mol/kg of calcium lactate or
(ii) 0,01 to 0,5 mol/kg of lactic acid (b1) and
0,05 to 0,5 mol/kg of calcium chloride and/or calcium hydroxide (b2)
b) at least one surfactant selected from anionic surfactants of the group formed from alkyl (ether) sulfates, sulfosuccinates, ether carboxylic acids, acyl glutamate and/or acyl isethionates each having 8 to 24 carbon atoms in the acyl group and mixtures of these surfactants, and/or
**characterized in that** the cleaning or care composition has a pH in the range of 4.2 to 4.7.

6. Cosmetic cleansing or conditioning composition according to claim 5, **characterized in that** it comprises at least one anionic surfactant in a proportion by weight of from 0.5 to 20%, preferably from 1 to 15% and in particular from 2 to 12%, the quantities being based on the total weight of the cleansing or conditioning composition.

7. Cosmetic cleansing or conditioning composition according to one of claims 5 or 6, **characterized in that** it comprises, by weight
- 0.06 to 0.30 mol/kg and in particular 0.07 to 0.20 mol/kg of calcium lactate or
- 0.02 to 0.40 mol/kg, preferably 0.03 to 0.30 mol/kg and in particular 0.05 to 0.20 mol/kg of lactic acid (b1) and 0.05 to 0.40 mol/kg, preferably 0.06 to 0.30 mol/kg and in particular 0.07 to 0.20 mol/kg of calcium chloride and/or calcium hydroxide (b2).

8. Cosmetic cleansing or conditioning composition according to any one of claims 5 to 7, **characterized in that** it additionally comprises at least one hair-conditioning active ingredient in a proportion by weight of 0.01 to 10% of the total weight of the cosmetic cleansing or conditioning composition.

9. Cosmetic cleansing or conditioning composition according to claim 8, **characterized in that** the at least one hair-conditioning active ingredient is selected from cationic polymers, in particular from cationic polysaccharides, and/or from vegetable oils and/or silicones.

10. Cosmetic cleansing or conditioning composition according to any one of claims 5 to 9, **characterized in that** it is substantially free of nitrogen-containing complexing and/or chelating agents.

11. Cleansing or conditioning cosmetic composition according to any one of claims 5 to 10, comprising by weight
a) 0.04 to 0.40 mol/kg, preferably 0.06 to 0.30 mol/kg and in particular 0.07 to 0.20 mol/kg of calcium lactate or
0.02 to 0.40 mol/kg, preferably 0.03 to 0.30 mol/kg and in particular 0.05 to 0.20 mol/kg of lactic acid and 0.05 to 0.40 mol/kg, preferably 0.06 to 0.30 mol/kg and in particular 0.07 to 0.20 mol/kg of calcium chloride or calcium hydroxide,
b) from 0.5 to 20% by weight, preferably from 1 to 15% and in particular from 2 to 12% by weight, of at least one anionic surfactant and/or from 0.01 to 5% by weight, preferably from 0.05 to 2% by weight and in particular from 0.1 to 0.5% by weight, of at least one cationic surfactant, and
c) 0.01 to 10% by weight, preferably 0.05 to 5% by weight and in particular 0.1 to 3% by weight of at least one cationic polysaccharide,
**characterized in that** the composition has a pH in the range of from 4.2 to 4.7, preferably from 4.25 to 4.65, and more preferably from 4.3 to 4.6.

12. Use of a cosmetic composition according to any one of claims 5 to 11 for the reduction and/or prevention of bleeding and/or fading of artificially generated hair colors.

## Revendications

1. Procédé pour réduire et/ou prévenir le saignement et/ou l'affadissement des colorants capillaires produits artificiellement, comprenant les étapes suivantes :
i. application d'une composition cosmétique sur les cheveux colorées, de préférence humides,
ii. laissez la composition agir pendant une période d'au moins 5 secondes,
iii. rincez la composition à l'eau,
**caractérisé en ce que**
- la composition cosmétique - par rapport à son poids - est
a) 0,05 à 0,5 mol/kg de lactate de calcium ou
b) 0,01 à 0,5 mol/kg d'acide lactique (b1) et 0,05 à 0,5 mol/kg de chlorure de calcium et/ou d'hydroxyde de calcium (b2), et
- **en ce que** la composition cosmétique a un pH compris entre 4,0 et 4,8.

2. Procédé selon la revendication 1, **caractérisé en ce que** la composition cosmétique - par rapport à son poids - comprend
- 0,06 à 0,30 mol/kg et en particulier 0,07 à 0,20 mol/kg de lactate de calcium ou
- 0,02 à 0,40 mol/kg, de préférence 0,03 à 0,30 mol/kg et en particulier 0,05 à 0,20 mol/kg d'acide lactique (b1) et 0,05 à 0,40 mol/kg, de préférence 0,06 à 0,30 mol/kg et en particulier 0,07 à 0,20 mol/kg de chlorure de calcium et/ou d'hydroxyde de calcium (b2).

3. Procédé selon l'une quelconque des revendications précédentes, **caractérisé en ce que** le pH de la composition cosmétique est compris entre 4,1 et 4,75, de préférence entre 4,2 et 4,7 et en particulier entre 4,3 et 4,6.

4. Procédé selon l'une quelconque des revendications précédentes, **caractérisé en ce que** les cheveux ont été colorées avec des agents de coloration des cheveux oxydants.

5. Composition cosmétique nettoyante ou conditionnante pour réduire et/ou prévenir le saignement et/ou la décoloration des colorants capillaires produits artificiellement, contenant par rapport à son poids
a)
(i) 0,05 à 0,5 mol/kg de lactate de calcium ou
(ii) 0,01 à 0,5 mol/kg d'acide lactique (b1) et
0,05 à 0,5 mol/kg de chlorure de calcium et/ou d'hydroxyde de calcium (b2)
b) au moins un agent tensioactif choisi parmi les tensioactifs anioniques du groupe formé par les (éther)sulfates d'alkyle, les sulfosuccinates, les acides éthercarboxyliques, les glutamates d'acyle et/ou les iséthionates d'acyle ayant chacun 8 à 24 atomes de carbone dans le groupe acyle et les mélanges de ces agents tensioactifs, et/ou
**caractérisé en ce que** la composition de nettoyage ou de conditionnante a un pH dans la gamme de 4,2 à 4,7.

6. Composition cosmétique de nettoyage ou de conditionnante selon la revendication 5, **caractérisée en ce qu'**elle contient au moins un tensioactif anionique dans une proportion pondérale de 0,5 à 20%, de préférence de 1 à 15% et en particulier de 2 à 12%, les quantités étant rapportées au poids total de la composition de nettoyage ou de conditionnante.

7. Composition cosmétique de nettoyage ou de conditionnante selon l'une des revendications 5 ou 6, **caractérisée en ce qu'**elle comprend, en poids
- 0,06 à 0,30 mol/kg et en particulier 0,07 à 0,20 mol/kg de lactate de calcium ou
- 0,02 à 0,40 mol/kg, de préférence 0,03 à 0,30 mol/kg et en particulier 0,05 à 0,20 mol/kg d'acide lactique (b1) et 0,05 à 0,40 mol/kg, de préférence 0,06 à 0,30 mol/kg et en particulier 0,07 à 0,20 mol/kg de chlorure de calcium et/ou d'hydroxyde de calcium (b2).

8. Composition cosmétique de nettoyage ou de conditionnante selon l'une quelconque des revendications 5 à 7, **caractérisée en ce qu'**elle contient en outre au moins un actif de conditionnement des cheveux dans une proportion pondérale de 0,01 à 10% du poids total de la composition cosmétique de nettoyage ou de conditionnante.

9. Composition cosmétique de nettoyage ou de conditionnante selon la revendication 8, **caractérisée en ce que** l'au moins un actif de conditionnement des cheveux est choisi parmi les polymères cationiques, en particulier parmi les polysaccharides cationiques, et/ou parmi les huiles végétales et/ou les silicones.

10. Composition cosmétique de nettoyage ou de conditionnement selon l'une quelconque des revendications 5 à 9, **caractérisée en ce qu'**elle est sensiblement exempte d'agents complexants et/ou chélateurs azotés.

11. Composition cosmétique de nettoyage ou de conditionnante selon l'une quelconque des revendications 5 à 10, comprenant, par rapport à son poids
a) 0,04 à 0,40 mol/kg, de préférence 0,06 à 0,30 mol/kg et en particulier 0,07 à 0,20 mol/kg de lactate de calcium ou
0,02 à 0,40 mol/kg, de préférence 0,03 à 0,30 mol/kg et en particulier 0,05 à 0,20 mol/kg d'acide lactique et 0,05 à 0,40 mol/kg, de préférence 0,06 à 0,30 mol/kg et en particulier 0,07 à 0,20 mol/kg de chlorure de calcium ou d'hydroxyde de calcium,
b) 0,5 à 20% en poids, de préférence de 1 à 15 et en particulier de 2 à 12% en poids d'au moins un agent tensioactif anionique et/ou 0,01 à 5% en poids, de préférence de 0,05 à 2% en poids et en particulier de 0,1 à 0,5% en poids d'au moins un agent tensioactif cationique, et
c) 0,01 à 10% en poids, de préférence 0,05 à 5% en poids et en particulier 0,1 à 3% en poids d'au moins un polysaccharide cationique,
**caractérisé en ce que** la composition a un pH dans la gamme de 4,2 à 4,7, de préférence de 4,25 à 4,65 et plus préférablement de 4,3 à 4,6.

12. Utilisation d'une composition cosmétique selon l'une quelconque des revendications 5 à 11 pour la réduction et/ou prévention des saignements et/ou de l'affadissement des colorants capillaires produits artificiellement.
